(19) Europäisches Patentamt
European Patent Office
Office européen des brevets

(11) Numéro de publication: **0 412 880 B1**

# (12) FASCICULE DE BREVET EUROPEEN

(45) Date de publication du fascicule du brevet: **22.11.95**

(51) Int. Cl.[6]: **C12P 17/02**, C07D 307/32, C07D 309/30

(21) Numéro de dépôt: **90402217.5**

(22) Date de dépôt: **02.08.90**

(54) **Procédé de production microbiologique des gamma- et delta-lactones.**

(30) Priorité: **04.08.89 IT 6768889**

(43) Date de publication de la demande:
**13.02.91 Bulletin 91/07**

(45) Mention de la délivrance du brevet:
**22.11.95 Bulletin 95/47**

(84) Etats contractants désignés:
**AT BE CH DE DK ES FR GB GR IT LI LU NL SE**

(56) Documents cités:
**EP-A- 258 993**
**EP-A- 0 356 291**
**IT-A- 6 774 288**
**US-A- 4 560 656**
**EP-A- 371 568**
**WO-A-83/01072**
**US-A- 4 396 715**

(73) Titulaire: **PERNOD-RICARD**
**142, Boulevard Haussmann**
**F-75008 Paris (FR)**

(72) Inventeur: **Cardillo, Rosanna**
**Via Pietro da Cortona 7**
**Milan (IT)**
Inventeur: **Fuganti, Claudio**
**Via G.V. Nazari 8**
**Milan (IT)**
Inventeur: **Barbeni, Massimo**
**Via Baretti 19**
**Turin (IT)**
Inventeur: **Cabella, Paolo**
**Corso Sommellier 17**
**Turin (IT)**
Inventeur: **Guarda, Pier Antonio**
**Regione Mandria 20**
**I-10040 Druento Turin (IT)**
Inventeur: **Gianna, Allegrone**
**Via Tofane 44**
**I-10141 Turin (IT)**

(74) Mandataire: **Schrimpf, Robert et al**
**Cabinet Regimbeau**
**26, Avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un procédé microbiologique pour la préparation de gamma- ou delta-lactones selon la formule générale :

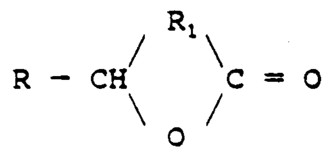

(I)

dans laquelle R est une chaîne alkylique linéaire saturée, mono-, di- ou tri-insaturée comprenant de 2 à 10 atomes de carbone et $R_1$ est alkylène comprenant 2 ou 3 atomes de carbone.

De nombreuses lactones de la formule générale (I) citée ci-dessus sont présentes dans de nombreux aliments et, par conséquent, jouent un rôle très important dans l'industrie des arômes. De telles lactones sont décrites, par exemple, par S. Arctander dans "Perfume and Flavor Chemicals", vol. I et II, Montclair, N.J. (USA), 1969.

Ces substances sont généralement dotées d'une activité optique, mais, comme il résulte de récentes études analytiques (cfr. Schuring, Bioflavour 1987, P. Schreier Ed.; de Gruyter, Berlin, 1988, page 35; A. Mosandl et al., *ibidem* page 55), la pureté optique et la configuration absolue d'un même composé peuvent varier en fonction de la source naturelle de laquelle il a été isolé.

Les composés indiqués ci-dessus sont importants du point de vue industriel, mais l'isolement des sources naturelles n'est pas praticable d'un point de vue économique, étant donné la faible concentration à laquelle ils sont présents à l'état naturel.

En revanche, la production par dégradation microbiologique de précurseurs biosynthétiques avancés est plus avantageuse. A cet effet, le brevet USA n° 4 560 656 décrit un procédé pour la production du (R) gamma-décanolide à partir d'acide ricinoléique naturel lorsqu'il est mis en contact avec des micro-organismes appartenant pour la plupart au *Candida*. La demande de brevet EP-A-258 993 décrit la production d'un gamma-décanolide optiquement actif, par culture des micro-organismes *Sporobolomyces odorus* et *Rhodotorula glutinis*. D'autres micro-organismes capables de produire le (R) gamma-décanolide et le (R) gamma-octanolide dans un milieu de culture comprenant une huile végétale sont décrits dans la demande de brevet italienne n° 67742- A/88 publiée le 5 février 1990 et équivalente à la demande EP-A 0 356 291 qui s'avère comprise dans l'état de la technique tel que défini à l'article 54(3) CBE.

Conformément à la présente invention, on a obtenu des cultures de micro-organismes qui sont capables de dégrader en lactones conformes à la formule générale (I) citée ci-dessus les hydroxydes ou hydroperoxydes d'acides linéaires insaturés naturels ayant un carboxyle soit libre, soit estérifié, sous une forme optiquement active ou racémique.

L'objet de la présenta invention est donc un procédé pour la préparation de gamma- ou delta-lactones selon la formule générale (I), dans laquelle R et $R_1$ ont la signification définie plus haut comportant l'opération consistant à cultiver, dans un substrat contenant un composé choisi dans le groupe constitué des hydroxydes et hydroperoxydes d'acide linoléique et d'acide linolénique, de leurs esters avec les alcools inférieurs en $C_1$-$C_4$, de leurs glycérides et des mélanges de tels composés, un micro-organisme capable d'effectuer une oxydation en bêta desdits composés.

Les hydroxydes et hydroperoxydes d'acide linoléique et d'acide linolénique utiles pour la mise en oeuvre de la présente invention sont illustrés au tableau I qui suit, accompagnés des lactones que l'on peut obtenir à la suite de leur dégradation par voie microbiologique.

## TABLEAU I

### HYDROXYDES ET HYDROPEROXYDES D'ACIDE LINOLEIQUE ET D'ACIDE LINOLENIQUE ET LACTONES POUVANT EN RESULTER.

| | DERIVE | R | LACTONE |
|---|---|---|---|
| (1) | 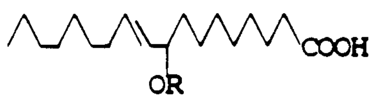 | OH | 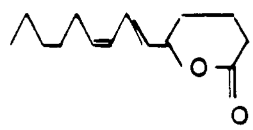 |
| (2) | idem | H | idem |
| (3) | 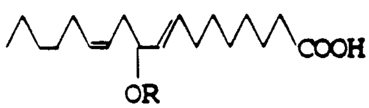 | OH | 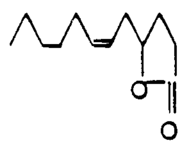 |
| (4) | idem | H | idem |
| (5) | 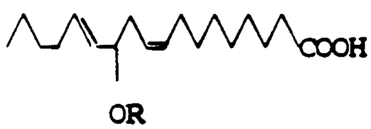 | OH | 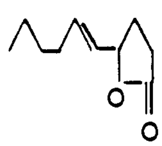 |
| (6) | idem | H | idem |
| (7) | 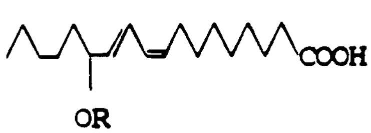 | OH | 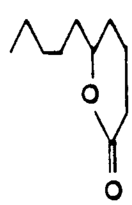 |
| (8) | idem | H | idem |

| | | | |
|---|---|---|---|
| (9) | 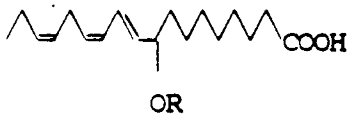 | OH | 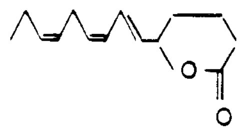 |
| (10) | idem | H | idem |
| (11) | 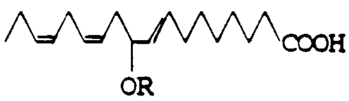 | OH | 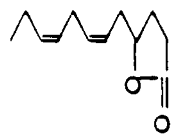 |
| (12) | idem | H | idem |
| (13) | 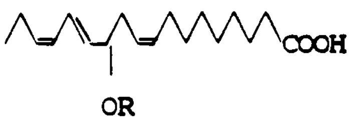 | OH | 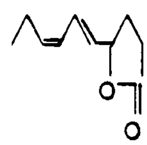 |
| (14) | idem | H | idem |
| (15) | 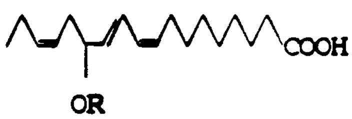 | OH | 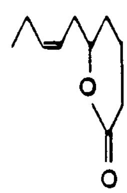 |
| (16) | idem | H | idem |
| (17) | 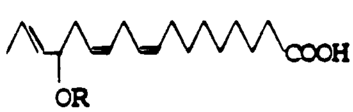 | OH | 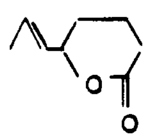 |

| | | | |
|---|---|---|---|
| (18) | idem | H | idem |
| (19) | 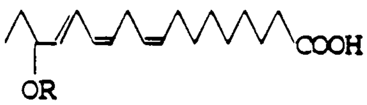 | OH | 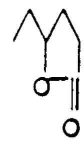 |
| (20) | idem | | idem |

De tels composés peuvent être utilisés dans le cadre du procédé, soit sous une forme racémique, soit sous une forme optiquement active.

De tels composés peuvent être obtenus à partir de matières premières facilement disponibles selon des procédés connus. En particulier, des mélanges d'hydroperoxydes sous une forme racémique sont obtenus par photo-oxygénation d'acide linoléique ou d'acide linolénique, libre ou estérifié, avec des alccols inférieurs de 1-4 atomes de carbone ou des glycérides qui les contiennent. La photo-oxygénation est effectuée dans un solvant approprié en présence d'un photoactivateur naturel, comme décrit amplement dans la technique. A ce propos, se référer au texte "The Lipid Handbook", Chapman & Hall, Londres, 1986, page 453.

Les hydroperoxydes des formules (1) et (7) (tableau I) sont accessibles sous une forme optiquement active par lipo-oxygénation, au moyen de lipo-oxygénases naturelles. En particulier, l'utilisation de lipo-oxygénases de soja permet d'obtenir en mode exclusif l'hydroperoxyde de la formule (7), avec la configuration (S) (H. D. Belitz & W. Grosch, Food Chemistry, Springer Verlag, 1987, pages 164-171).

Dans le cadre de la présente invention, l'utilisation d'hydroxydes est préférée à celle des hydroperoxydes correspondants. En particulier, les hydroxydes racémiques peuvent être obtenus à partir de mélanges des hydroperoxydes correspondants par traitement avec un réducteur naturel, par exemple le sulfate ferreux ou la cystéine dans le solvant approprié selon la technique décrite par H. D. Belitz & W. Grosch dans Food Chemistry, Springer Verlag, Berlin, 1987, page 172 et H. W. Gardner, J. Agr. Food Chem., 1975, 23, 129.

L'hydroxyde de la formule (8) du tableau I sous les deux formes énantiomères (S) et (R) est isolé à partir de plantes différentes, alors que la forme racémique a également été isolée de sources naturelles.

En particulier, l'acide (-)coriolique est isolé de Coriaria nepalensis (13R) par J. H. Tallent dans Tetr. Letters, 1966, 4329; l'acide (+)coriolique (13S) de Mannina emarginata, dans R. C. Powel et al. dans J. Org. Chem., 1967, 32, 1442 et sous forme racémique, d'huile d'absinthe essentielle, dans The Lipids Handbook, Chapman et al., Londres, 1986, 133 .

Les hydroxydes et hydroperoxydes peuvent être soumis séparément aux micro-organismes sous la forme d'acides libres ou estérifiés ou de glycérides, ou en mélange de deux ou de plusieurs produits. Dans le premier cas, on obtiendra une seule lactone et, dans les autres, des mélanges de produits.

Les micro-organismes préférés utilisables dans le cadre de la présente invention sont choisis dans le groupe constitué de Cladosporium suaveolens, Cladosporium cucumberinum, Pichia etchellsii, Sporobolomyces salmonicolor, Candida lipolytica et Fusarium poae. Rhodotorula glutinis, Kloeckera saturnus, Sporobolomyces roseus, Cladosporium capsici, Pichia membranaefaciens, Pichia pamtoris, Hyphopichia burtoni, Kluyveromyces lactis, Aspergillus oryzae, Geotricum klebahnii, Saccharomyces cerevisiae, Saccharomyces delbrueckii and Monilinia fructicola. Ces micro-organismes sont disponibles auprès des centres de récolte.

D'autres micro-organismes capables d'effectuer l'oxydation en bêta sont décrits dans le brevet USA n° 4 560 656 et dans le brevet EP-A-258 993.

Selon un autre aspect, l'invention fournit un procédé pour la préparation d'une gamma- ou delta-lactone selon la formule générale (I) citée ci-dessus, dans laquelle R est une chaîne alkylique linéaire saturée ou mono-insaturée comprenant de 8 à 10 atomes de carbone et dans laquelle $R_1$ est alkylène comprenant 2 ou 3 atomes de carbone, comportant l'opération consistant à cultiver, dans un substrat comprenant un produit choisi dans le groupe constitué :

- du produit de la photo-oxygénation de l'acide oléique, d'un de ses esters avec les alcools inférieurs en $C_1$-$C_4$ et de ses glycérides,
- du produit de l'auto-oxydation de matières grasses comprenant de l'acide oléique, un de ses esters avec des alcools inférieurs en $C_1$-$C_4$ et ses glycérides,

un micro-organisme capable d'effectuer l'oxydation en bêta dudit produit.

Les produits précités de la photo-oxygénation et de l'auto-oxydation comprennent des mélanges d'isomères d'hydroperoxydes d'acide oléique contenant en majeure partie le composé présentant une double liaison à la position 10 et le groupe hydroperoxydes à la position 9, ainsi que le composé présentant une double liaison à la position 8 et le groupe hydroperoxydes à la position 10.

Les hydroxydes correspondants peuvent être obtenus par réduction des produits précités selon la manière décrite précédemment.

En particulier, il est possible d'obtenir un bon rendement en gamma-dodécanolide à partir de dérivés d'acide oléique.

Les micro-organismes utilisables selon cette forme de mise en oeuvre sont les mêmes que ceux décrits précédemment.

La lactone désirée est obtenue à partir des précurseurs cités plus haut dans des conditions de croissance variables et dans des délais relativement courts, compris entre 24 et 60 heures. De manière typique, les micro-organismes sont maintenus en contact avec les matières indiquées plus haut à des températures comprises entre 20 et 30 ° C.

L'agent nutritif dans lequel s'effectue la croissance préalable des micro-organismes est du type classique. De préférence, la culture se déroule avec agitation dans la phase de dégradation qui conduit à la production de la lactone, alors que la production de la biomasse utilisée comme agent de préinoculation peut être effectuée soit en phase stationnaire, soit avec agitation.

On a en outre découvert que le rendement de production des gamma- et delta-lactones, dans le procédé selon l'invention, peut être grandement amélioré grâce à l'addition au substrat, tel que défini dans la présente description, d'acide ricinoléique ou de ses esters. On sait que plusieurs microorganismes, notamment ceux décrits en rapport avec la présente invention, sont capables d'effectuer une bêta-oxydation de l'acide ricinoléique ou de ses esters; cependant, le produit de la bêta-oxydation est le gamma-décanolide, qui est, en revanche, produit en quantité très limitée au moyen du substrat selon cette invention.

On a découvert que l'addition au substrat d'acide ricinoléique ou de ses esters a un effet stimulant sur l'activité des microorganismes que l'on a cités ci-dessus dans la production des gamma- et des delta-lactones de formule (I). L'addition d'acide ricinoléique ne semble pas influer sur la cinétique du procédé microbiologique, mais seulement sur l'activité. Autrement dit, on obtient le rendement maximum dans les mêmes délais que ceux nécessaires pour obtenir le rendement maximum en l'absence d'acide ricinoléique ou de ses esters.

De préférence, on ajoute l'acide ricinoléique ou ses esters au substrat dans un rapport allant de 1:2 à 2:1 et les microorganismes préférés dans ce mode de réalisation sont Candida lipolytica et Rhodotorula glutinis.

Comme il est connu, la forme lactonique du composé souhaité est sujette à une isomérisation avec la forme hydroxyacide correspondante et l'extraction de la lactone implique donc la transformation de l'hydroxyacide en la lactone correspondante.

Pour l'extraction de la lactone, on procède par filtration du bouillon de culture sur de la Celite® qui est lavée avec de l'acétate d'éthyle, puis on soumet la phase aqueuse de pH acide, de préférence pH 5, à l'extraction à l'acétate d'éthyle, au moins deux fois dans la mesure possible. Les phases organiques réunies sont extraites deux fois avec de la solution à 5% de carbonate de potassium pour éliminer les composants acides. Par la suite, la phase organique, séchée sur du sulfate de sodium, est évaporée et le résidu est distillé à 150 ° C sous 3 mm Hg pour obtenir le composé sous la forme lactonique.

Pour améliorer le rendement en lactone, il est possible de transformer en lactone l'acide correspondant en portant le pH entre 1 et 5, de préférence entre 1 et 3, en ajoutant un acide approprié et en réchauffant le milieu acidifié à une température comprise entre 50 et 110 ° C, de préférence comprise entre 90 et 100 ° C, pendant une période allant d'environ 10 minutes à 2 heures en fonction de la température. On peut séparer la lactone du milieu par entraînement à la vapeur à partir de ce milieu acidifié.

Dans les exemples suivants, les quantités de la lactone produite sont exprimées en pour-cent résultant d'analyses CGL. Lorsqu'elle est signalée, la chiralité des composants obtenus a été déterminée par la méthode décrite par M. Gessner et al. dans Z. Lebensm Unter Forsch, 1988, 186, 417.

EXEMPLE 1.-

On soumet 1 g d'un mélange d'acides hydroxylinoléiques à des cultures de Cladosporium suaveolens. On extrait le bouillon de culture après un jour, puis on élimine le solvant et on obtient une huile brute contenant :

| | Pourcentage par CGL |
|---|---|
| gamma-5-décénolide | 5,2 |
| gamma-décanolide | 1,0 |
| delta-décanolide | 6,7 |
| gamma-6-dodécénolide | 6,3 |

EXEMPLE 2.-

On soumet 1 g d'un mélange d'acides hydroxyoléiques à des cultures de Cladosporium suaveolens. On obtient une huile brute comprenant 2,4% de gamma-dodécénolide.

EXEMPLE 3.-

On soumet 300 g d'un mélange d'acides hydroxylinoléniques à une culture de 300 ml de Cladosporium s. Le mélange utilisé est le produit de la photo-oxygénation d'acide linolénique réduit avec des ions $Fe^{2+}$ ou de la cystéine pour donner un mélange des hydroxydes des formules (10), (12), (14), (16), (18) et (20) du tableau I avec un rapport entre les constituants de 23:13:12:14:13:25.

Après 24 heures d'incubation, l'extrait a la composition suivante :

| | |
|---|---|
| gamma-hexanolide | 0,5% |
| delta-7-décénolide | 7,2% |
| gamma-6,9-dodécadiénolide | 9,0% |
| delta-octanolide | 0,9% |
| gamma-5-décénolide | 1,3% |
| gamma-5,7-décadiénolide | 3,4% |

EXEMPLE 4.-

On soumet 300 mg d'un mélange d'acides hydroxylinoléiques à une culture de 300 ml de Cladosporium suaveolens (5 g par litre d'extrait de viande). Le mélange utilisé, obtenu par photo-oxygénation de l'acide linoléique, puis réduction, comme décrit dans l'exemple 3, contient un mélange des hydroxydes (2), (4), (6) et (8) du tableau I dans un rapport de 32:17:17:32. Après 24 heures d'incubation, on obtient :

| | |
|---|---|
| gamma-5-décénolide | 11% |
| gamma-décanolide | 1% |
| delta-décanolide | 16% |
| gamma-6-dodécénolide | 20% |

EXEMPLE 5.-

Dans 17 matras contenant chacun 100 ml d'eau et 25 mg de mélange d'hydroxyperoxydes de l'acide linoléique, contenant les composés (1), (3), (5) et (7) dans un rapport de 32:17:17:34, on ajoute une suspension aqueuse de cellules de Cladosporium suaveolens obtenue dans des éprouvettes. Après 24 heures, les extraits réunis concentrés donnent 70 mg d'huile brute contenant :

| | |
|---|---|
| gamma-5-décénolide | 0,8% |
| delta-décanolide | 2,6% |
| gamma-6-dodécénolide | 0,7% |

EXEMPLE 6.-

La procédure de l'exemple 5 est répétée tout en ajoutant dans chacun des matras 100 mg d'un mélange d'esters méthyliques d'acides hydroxylinoléiques.

Après 48 heures d'incubation, les extraits réunis et concentrés donnent une huile brute contenant :

| | |
|---|---|
| gamma-5-décénolide | 7,8% |
| delta-décanolide | 4,3% |
| gamma-6-dodécénolide | 28% |

EXEMPLE 7.-

On ajoute dans chacun des 10 matras contenant 100 ml de culture de Cladosporium suaveolens 100 mg d'un mélange d'esters méthyliques des acides hydroxylinoléiques (ensemencement directement dans l'eau distillée à pH 7).

Après 7 jours, les extraits réunis et concentrés donnent une huile brute contenant :

| | |
|---|---|
| gamma-5-décénolide [dont 40% d'isomère (R) et 60% d'isomère (S)] | 5,1% |
| gamma-6-dodécénolide [dont 53% d'isomère (R) et 47% d'isomère (S)] | 18,5% |
| gamma-décanolide [dont 7% d'isomère (R) et 93% d'isomère (S)] | 1,2% |

EXEMPLE 8.-

On incube 3 matras de 100 ml de culture de Pichia e. inoculée avec un mélange de 300 mg d'hydroxydes racémiques de l'acide linoléique pendant 24 heures. L'extrait concentré et distillé dans une ampoule donne 20 mg d'huile brute contenant :

| | |
|---|---|
| gamma-5-décénolide | 13% |
| gamma-décanolide | 1,3% |
| gamma-6-dodécénolide | 2,2% |
| gamma-décanolide [dont 23% d'isomère (R) et 77% d'isomère (S)] | 22% |

EXEMPLE 9.-

On répète la procédure de l'exemple 8 en utilisant le micro-organisme Fusarium poae et on obtient après fermentation pendant 24 heures une huile brute contenant :

| | |
|---|---|
| gamma-hexanolide | 0,72% |
| gamma-décanolide | 1% |
| gamma-5-décénolide [dont 61% d'isomère (R) et 39% d'isomère (S)] | 2,9% |
| gamma-6-dodécénolide [dont 20% d'isomère (R) et 80% d'isomère (S)] | 2,8% |

EXEMPLE 10.-

On répète la procédure de l'exemple 8 en utilisant le micro-organisme Sporobolomyces salmonicolor. On utilise 300 mg d'acides hydroxylinoléiques séparés par chromatographie liquide en deux fractions A et B.

Après une incubation de trois jours, les huiles brutes présentant les concentrations suivantes sont obtenues :

| | Huile brute fraction A | Huile brute fraction B |
|---|---|---|
| gamma-nonanolide | 0,36% | 0,62% |
| gamma-5-décénolide | 2,4% | 0,34% |
| gamma-décanolide | 1,0% | 0,46% |
| delta-décanolide | 14,1% | 0,5% |
| gamma-6-dodécénolide | 7,12% | 0,92% |
| gamma-dodécanolide | 0,36% | - |

EXEMPLE 11.-

On opère selon la procédure de l'exemple 8 en utilisant Candida lipolytica et les acides hydroxylinoléiques séparés en deux fractions chromatographiques A et B, et on obtient les huiles brutes présentant les concentrations suivantes:

| | Huile brute fraction A | Huile brute fraction B |
|---|---|---|
| gamma-5-décénolide | 19,7% | 3,2% |
| delta-décanolide | 1,5% | 0,2% |
| gamma-6-dodécénolide | 0,66% | 0,2% |

EXEMPLE 12.-

On opère selon la procédure de l'exemple 8 en utilisant Cladosporium cucumberinum avec 300 mg d'acides hydroxylinoléiques séparés en fractions A et B, et, après sept jours, on obtient :

| | Huile brute fraction A | Huile brute fraction B |
|---|---|---|
| gamma-5-décénolide | 2,6% | 0,65% |
| gamma-décanolide | 1,07% | 0,33% |
| delta-décanolide | 1,15% | 0,12% |
| gamma-6-dodécénolide | 0,7% | 0,5% |

EXEMPLE 13.-

On introduit dans 750 ml de culture de Cladosporium suaveolens 2 g d'huile de tournesol photooxydée dans l'acétonitrile en présence d'un photoactivateur naturel (cercosporine), puis réduite (fraction chromatographique A). Après 48 heures, on obtient une huile brute contenant :

| | |
|---|---|
| gamma-5-décénolide | 1,7% |
| gamma-décanolide | 0,3% |
| gamma-6-dodécénolide | 4,1% |

La fraction B de l'huile de tournesol photooxydée et réduite donne dans des conditions analogues :

| | |
|---|---|
| gamma-5-décénolide [dont 49% d'isomère (R) et 51% d'isomère (S)] | 1,5% |
| gamma-décanolide | 0,3% |
| gamma-6-dodécénolide [dont 76% d'isomère (R) et 24% d'isomère (S)] | 2,1% |

EXEMPLE 14.-

On soumet 800 mg d'acide (R)-coriolique [acide (R)-13-hydroxy-cis-9,trans-11-octadécénoïque] à des cultures de Pichia e. et, après 24 heures, on obtient une huile brute contenant 35% de (R)-delta-décanolide.

EXEMPLE 15.-

On soumet à une culture de Pichia etchellsii 800 mg d'acide (S)-coriolique obtenus par lipo-oxygénation d'acide linoléique avec des lipo-oxygénases de soja (FLUKA) selon le procédé décrit par B. Axelrod dans Methods in Enzymology, 1981, page 441, puis par réduction avec des sels ferreux et, après 24 heures, on obtient une huile brute contenant 40% de (S)-delta-décanolide. Le même composé peut être produit à l'aide de Cladosporium s..

Le procédé conforme à l'invention fournit donc la possibilité d'obtenir des composés utiles dans l'industrie à partir de sources peu coûteuses dérivant des matières grasses qui n'ont plus aucune autre valeur alimentaire. Comme il résulte des exemples portant sur les micro-organismes examinés, le processus de dégradation de l'hydroxyde ou de l'hydroperoxyde sous la forme optiquement active est énantiosélectif, grâce à quoi il est possible de produire la lactone désirée avec une pureté énantiomère élevée.

Exemple 16

On soumet les esters méthyliques des acides contenus dans l'huile de tournesol, obtenus par transestérification avec du méthanol en présence de lipase microbienne, à une photooxydation en présence de cercosporine, comme substance photodynamique, et de lumière du soleil, en faisant passer de l'air dans la solution dans l'acétonitrile, On utilise 10 g des esters méthyliques indiqués ci-dessus dans 100 ml de $CH_3CN$ et 1 g de cercosporine avec la lumière du soleil et l'air.

A l'issue de la réaction, on concentre la solution des hydroperoxydes à un faible volume et on reprend le résidu avec de l'éthanol et on ajoute 3 g de sel de sodium de L-cistéine, tout en agitant vigoureusement. On poursuit l'agitation pendant deux heures et on extrait le mélange des esters méthyliques des acides gras hydroxylés avec de l'acétate d'éthyle. Le produit résultant est soumis à une hydrolyse avec de la lipase pour donner les hydroxyacides correspondants à pH 8,5 dans un système à deux phases constitué de chlorure de méthylène/eau. Les hydroxyacides sont récupérés dans la solution acide par extraction, suivie d'une élimination du solvant.

On inocule 500 mg des hydroxyacides dans 5 flacons, contenant chacun 100 ml d'une culture de Rhodotorula glutinis (extrait de viande 2 g/l)

Au bout de trente heures d'indubation à 30 °C, l'extrait a la composition suivante :

| | |
|---|---|
| gamma-5-décénolide | 4,5% |
| gamma-décanolide | 1,1% |
| delta-décanolide | 9,9% |
| gamma-6-dodécénolide | 5,5% |

Exemple 17

On reprend la procédure de l'exemple 16 en utilisant 5 flacons, contenant chacun 100 ml d'une culture de Kloeckera saturnus. La composition de l'extrait est la suivante :

| | |
|---|---|
| gamma-5-décénolide | 30% |
| gamma-décanolide | 1% |
| delta-décanolide | 3% |
| gamma-6-dodécénolide | 19% |

Exemple 18

On reprend la procédure de l'exemple 16, en utilisant 5 flacons, contenant chacun 100 ml de culture de Sporobolomyces roseus. L'extrait a la composition suivante :

| | |
|---|---|
| gamma-5-décénolide | 0,8% |
| gamma-décanolide | 3,9% |
| delta-décanolide | 0,4% |
| gamma-6-dodécénolide | 3,7% |
| gamma-dodécanolide | 2,3% |

Exemple 19

On reprend la procédure de l'exemple 16, en utilisant 5 flacons, contenant chacun 100 ml d'une culture de Cladosporium capsici. L'extrait a la composition suivante :

| | |
|---|---|
| gamma-5-décénolide | 1,1% |
| gamma-décanolide | 1,2% |
| delta-décanolide | 1,2% |

Exemple 20

On inocule 200 mg d'un mélange du produit obtenu par photooxygénation d'huile de pépin de raisin, selon la procédure de l'exemple 16, dans 100 ml d'une culture de Kluyveromyces lactis. Au bout de dix-huit heures, l'extrait obtenu a la composition suivante :

| | |
|---|---|
| gamma-5-décénolide | 0,9% |
| gamma-décanolide | 1,9% |
| gamma-6-dodécénolide | 8,9% |

Exemple 21

On applique la procédure de l'exemple 20 à des cultures de Hyphopichia burtoni. Au bout de dix-huit heures l'extrait a la composition suivante :

| | |
|---|---|
| gamma-5-décénolide | 24% |
| gamma-décanolide | 4% |
| delta-décanolide | 5% |
| gamma-6-dodécénolide | 28% |
| gamma-dodécanolide | 14% |

Exemple 22

On applique la procédure de l'exemple 20 à 3 flacons, contenant chacun 100 ml d'une culture de Geotricum klebahnii. Au bout de trente heures, l'extrait a la composition suivante :

| | |
|---|---|
| gamma-5-décénolide | 0,9% |
| delta-décanolide | 0,8% |
| gamma-6-dodécénolide | 0,9% |
| gamma-dodécanolide | 0,1% |

Exemple 23

On mélange une quantité, indiquée sur le tableau II, colonne 2, du substrat hydroxyacide obtenu selon l'exemple 16, avec une quantité, indiquée sur le tableau II, colonne 3, d'acide ricinoléique. Les mélanges sont mis en suspension dans 100 ml d'eau, contenant 2% en poids d'extrait de viande et 0,02% en poids de Tween. Les mélanges sont stérilisés et incubés avec les cultures de Candida lipolytica, obtenues à partir d'une culture en pente avec de l'eau distillée ou à partir d'un pré-inoculum. Les cultures sont agitées à 27-30°C pendant le temps indiqué sur le tableau I. A l'instant indiqué, on arrête la fermentation en amenant le pH à 2 et on effectue l'extration par solvant en ajoutant un étalon interne (gamma-$C_{11}$). Le solvant est évaporé et le résidu est distillé à 100-120°C sous un vide d'environ 0,1-0,3 mm Hg. Le produit résultant contenant les lactones recherchées est pesé et analysé. Les résultats obtenus avec différents rapports du substrat et de l'acide ricinoléique et différents temps sont indiqués sur le tableau II.

Exemple 24

On suit la procédure de l'exemple 23 en utilisant Rhodotorula glutinis.

Les résultats figurent au tableau III.

TABLEAU II

| t(n) | Substrat ml/100 ml | Acide ricinoléique ml/100 ml | $\gamma C_{10}\Delta 5$ mg/100 ml | $\delta C_{10}$ mg/100 ml | $\gamma C_{12}\Delta 6$ mg/100 ml | $\gamma C_{12}$ mg/100 ml | 1+2+3+4 mg/100 ml |
|---|---|---|---|---|---|---|---|
| 15 | 4 | 1 | 42 | 6,5 | 23,5 | 15 | 87 |
| 15 | 4 | 2 | 60 | 8 | 28,5 | 22 | 118,5 |
| 24 | 4 | 0 | 61,5 | 15,5 | 55 | 38 | 194 |
| 24 | 4 | 2 | 85,5 | 9,5 | 85 | 57 | 231 |
| 24 | 4 | 4 | 136,5 | 8 | 68 | 41 | 254 |
| 30 | 4 | 0 | 85 | 1,5 | 81 | 35 | 175 |
| 30 | 4 | 4 | 132,5 | - | 62 | 26 | 220 |
| 30 | 4 | 8 | 118,5 | 7 | 191 | 95,5 | 412 |

Les quantités de substrat et d'acide ricinoléique sont exprimées en ml/100 ml de culture et les quantités des lactones en ml/100 ml de culture

$\gamma C_{10}\Delta 5$ = gamma-5-décénolide

$\delta C_{10}$ = delta décanolide

$\gamma C_{12}\Delta 6$ = gamma-6-dodécénolide

$\gamma C_{12}$ = gamma-dodécanolide

TABLEAU III

| t(n) | Substrat ml/100 ml | Acide ricinoléique ml/100 ml | $\gamma C_{10}\Delta 5$ mg/100 ml | $\delta C_{10}$ mg/100 ml | $\gamma C_{12}\Delta 6$ mg/100 ml | $\gamma C_{12}$ mg/100 ml | 1+2+3+4 mg/100 ml |
|---|---|---|---|---|---|---|---|
| 12 | 4 | - | 15,5 | - | 9 | 7 | 31,5 |
| 12 | 4 | 8 | 30 | 0.25 | 38 | 32,5 | 101 |
| 48 | 4 | - | 2,1 | - | 2,7 | - | 5 |
| 48 | 4 | 8 | 12 | - | 9,5 | 7 | 28,5 |
| 48 | 8 | 4 | 27 | - | 18 | 18,5 | 63.5 |
| 55 | 8 | 8 | 1,5 | - | 103 | 45 | 149,5 |
| 144 | 8 | 4 | 110 | - | 64 | 53,5 | 227,5 |
| 144 | 8 | 4 | 135 | - | 146 | 48,5 | 329,5 |

## Revendications

**1.** Procédé pour la préparation d'une gamma- ou d'une delta-lactone selon la formule générale :

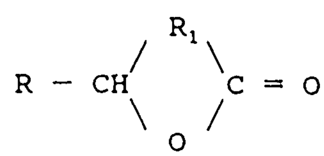

(I)

dans laquelle R est une chaîne alkylique linéaire saturée, mono-, di- ou tri-insaturée comprenant de 2 à 10 atomes de carbone et $R_1$ est alkylène comprenant 2 ou 3 atomes de carbone, comportant l'opération consistant à cultiver, dans un substrat contenant un composé choisi dans le groupe constitué des hydroxydes ou hydroperoxydes d'acide linoléique et d'acide linolénique, d'un de leurs esters avec les alcools inférieurs en $C_1$-$C_4$, de leurs glycérides et de leurs mélanges, un micro-organisme capable d'effectuer une oxydation en bêta dudit composé.

**2.** Procédé suivant la revendication 1, dans lequel le substrat est le produit de la photo-oxygénation de l'acide linoléique ou linolénique, libre ou estérifié, avec des alcools inférieurs en $C_1$-$C_4$ et des glycérides qui les contiennent.

**3.** Procédé suivant la revendication 1, dans lequel ledit substrat est le produit de l'auto-oxydation des matières grasses contenant de l'acide linoléique ou linolénique.

**4.** Procédé suivant la revendication 1, dans lequel ledit substrat est le produit de la lipo-oxygénation de l'acide linoléique ou linolénique ou des mélanges qui les contiennent avec des lipo-oxygénases naturelles.

**5.** Procédé pour la préparation d'une gamma- ou delta-lactone suivant la formule générale (I) selon la Revendication 1, dans lequel ledit substrat comprend un hydroxyde d'acide linoléique ou linolénique obtenu par réduction des produits définis dans l'une quelconque des revendications 2 à 4.

**6.** Procédé pour la préparation d'une gamma- ou delta-lactone suivant la formule générale (I), dans laquelle R est une chaîne alkylique linéaire saturée ou mono-insaturée comprenant de 8 à 10 atomes de carbone et dans laquelle $R_1$ est alkylène comprenant 2 ou 3 atomes de carbone, comportant l'opération consistant à cultiver dans un substrat comprenant le produit choisi dans le groupe constitué :

- du produit de la photo-oxygénation de l'acide oléique, d'un de ses esters avec les alcools inférieurs en $C_1$-$C_4$ et de ses glycérides,
- du produit de l'auto-oxydation de matières grasses comprenant de l'acide oléique, ses esters et les glycérides qui le contiennent,

un micro-organisme capable d'effectuer l'oxydation en bêta dudit produit.

**7.** Procédé suivant la revendication 6, dans lequel ledit substrat comprend un hydroxyde d'acide oléique obtenu par réduction des hydroxydes ou hydroperoxydes d'acide linoléique et d'acide linolénique, d'un de leurs esters avec les alcools inférieurs en $C_1$ - $C_4$, de leurs glycérides et de leurs mélanges.

**8.** Procédé suivant l'une quelconque des revendications 1 à 7, dans lequel ledit micro-organisme est choisi dans le groupe constitué de Cladosporium suaveolens, Cladosporium cucumberinum, Pichia etchellsii, Sporobolomyces salmonicolor, Candida lipolytica et Fusarium poae, Rhodotorula glutinis, Kloeckera saturnus, Sporobolomyces roseus, Cladosporium capsici, Pichia membranaefaciens, Pichia pamtoris, Hyphopichia burtoni, Kluyveromyces lactis, Aspergillus oryzae, Geotricum klebahnii, Saccharomyces cerevisiae, Saccharomyces delbrueckii and Monilinia fructicola.

**9.** Procédé suivant la revendication 1 pour la préparation de (S)-delta-décanolides, dans lequel ledit substrat comprend l'acide (S)-13-hydroxy (ou hydroperoxy)-cis-9, trans-11-octadécénoïque et dans lequel le micro-organisme employé est choisi dans le groupe constitué de Pichia etchellsii et de Cladosporium suaveolens.

**10.** Procédé suivant la revendication 9, dans lequel ledit hydroxyde ou hydroperoxyde est obtenu par lipo-oxygénation de l'acide linoléique avec des lipo-oxygénases de soja.

**11.** Procédé suivant l'une quelconque des revendications 1 à 10, dans lequel ledit hydroxyde ou hydroperoxyde se présente sous une forme optiquement active.

**12.** Procédé suivant l'une quelconque des revendications 1 à 11, dans lequel l'acide ricinoléique ou son ester est ajouté au substrat.

**13.** Procédé suivant la revendication 12, dans lequel le rapport entre le substrat et la quantité d'acide ricinoléique et de son ester est dans la gamme de 1:2 à 2:1.

**14.** Procédé suivant la revendication 12, dans lequel le microorganisme est choisi dans le groupe constitué par Candida lipolytica et Rhodotorula glutinis.

**Claims**

**1.** Process for the preparation of a gamma- or a delta-lactone according to the general formula:

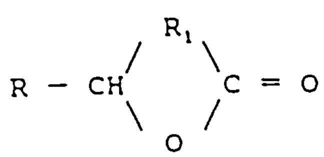

(I)

in which R is a saturated or mono-, di- or triunsaturated linear alkyl chain comprising from 2 to 10 carbon atoms and $R_1$ is alkylene comprising 2 or 3 carbon atoms, entailing the operation which consists in culturing, in a substrate containing a compound selected from the group consisting of the hydroxides or hydroperoxides of linoleic acid and of linolenic acid, of one of their esters with $C_1$-$C_4$ lower alcohols, of their glycerides and of mixtures thereof, a microorganism capable of performing a beta-oxidation of the said compound.

**2.** Process according to Claim 1, in which the substrate is the product of the photooxygenation of linoleic or linolenic acid, free or esterified with $C_1$-$C_4$ lower alcohols, and of glycerides which contain them.

**3.** Process according to Claim 1, in which the said substrate is the product of the autoxidation of fats containing linoleic or linolenic acid.

4. Process according to Claim 1, in which the said substrate is the product of the lipoxygenation of linoleic or linolenic acid or of mixtures which contain them with natural lipoxygenases.

5. Process for the preparation of a gamma- or delta-lactone according to the general formula (I) according to Claim 1, in which the said substrate comprises a hydroxide of linoleic or linolenic acid obtained by reduction of the products defined in any one of Claims 2 to 4.

6. Process for the preparation of a gamma- or delta-lactone according to the general formula (I), in which R is a saturated or monounsaturated linear alkyl chain comprising from 8 to 10 carbon atoms and in which $R_1$ is alkylene comprising 2 or 3 carbon atoms, entailing the operation which consists in culturing, in a substrate comprising the product selected from the group consisting of:
- the product of the photooxygenation of oleic acid, of one of its eaters with $C_1$-$C_4$ lower alcohols and of its glycerides and,
- the product of the autoxidation of fats comprising oleic acid, its eaters and glycerides which contain it, a microorganism capable of performing the beta-oxidation of the said product.

7. Process according to Claim 6, in which the said substrate comprises a hydroxide of oleic acid obtained by the reduction of the hydroxides or hydroperoxides of linoleic acid and of linolenic acid, of one of their eaters with $C_1$-$C_4$ lower alcohols, of their glycerides and of mixtures thereof.

8. Process according to any one of Claims 1 to 7, in which the said microorganism is selected from the group consisting of Cladosporium suaveolens, Cladosporium cucumberinum, Pichia etchellsii, Sporobolomyces salmonicolor, Candida lipolytica and Fusarium poae, Rhodotorula glutinis, Kloeckera saturnus, Sporobolomyces roseus, Cladosporium capsici, Pichia membranaefaciens, Pichia pamtoris, Hyphopichia burtoni, Kluyveromyces lactis, Aspergillus oryzae, Geotricum klebahnii, Saccharomyces cerevisiae, Saccharomyces delbrueckii and Monilinia fructicola. .

9. Process according to Claim 1 for the preparation of (S)-delta-decanolides, in which the said substrate comprises (S)-13-hydroxy(or hydroperoxy)-cis-9, trans-11-octadecenoic acid and in which the microorganism employed is selected from the group consisting of Pichia etchellsii and Cladosporium suaveolens.

10. Process according to Claim 9, in which the said hydroxide or hydroperoxide is obtained by the lipoxygenation of linoleic acid with soya bean lipoxygenases.

11. Process according to any one of Claims 1 to 10, in which the said hydroxide or hydroperoxide occurs in an optically active form.

12. Process according to any one of Claims 1 to 11, in which ricinoleic acid or its ester is added to the substrate.

13. Process according to Claim 12, in which the ratio of the substrate to the quantity of ricinoleic acid and of its ester is within the range 1:2 to 2:1.

14. Process according to Claim 12, in which the microorganism is chosen from the group consisting of Candida lipolytica and Rhodotorula glutinis.

**Patentansprüche**

1. Verfahren zur Herstellung eines - oder eines δ-Lactons der allgemeinen Formel:

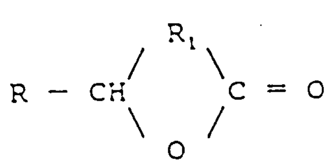

(I)

worin R eine lineare gesättigte, einfach-, zweifach- oder dreifach-ungesättigte Alkylkette mit 2 bis 10 Kohlenstoffatomen und $R_1$ Alkylen mit 2 oder 3 Kohlenstoffatomen bedeuten,
das eine Stufe umfaßt, die darin besteht, daß man in einem Substrat, das eine Verbindung, ausgewählt aus der Gruppe, die besteht aus Hydroxiden oder Hydroperoxiden von Linolsäure und Linolensäure, einem ihrer Ester mit niederen $C_1$-$C_4$-Alkoholen ihren Glyceriden und ihren Mischungen, enthält, einen Mikroorganismus kultiviert (züchtet), der in der Lage ist, eine Oxidation der genannten Verbindung in $\beta$-Stellung zu bewirken.

**2.** Verfahren nach Anspruch 1, worin das Substrat das Produkt der Photo-Oxidation der Linolsäure oder Linolensäure, die frei oder verestert ist mit niederen $C_1$-$C_4$-Alkoholen und der Glyceride, die sie enthalten.

**3.** Verfahren nach Anspruch 1, worin das genannte Substrat das Produkt der Auto-Oxidation von Fetten ist, die Linolsäure oder Linolensäure enthalten.

**4.** Verfahren nach Anspruch 1, worin das genannte Substrat das Produkt der Lipo-Oxidation der Linolsäure oder Linolensäure oder der Mischungen, die sie enthalten, mit natürlichen Lipooxygenasen ist.

**5.** Verfahren zur Herstellung eines - oder $\delta$-Lactons der allgemeinen Formel (I) nach Anspruch 1, worin das genannte Substrat ein Linolsäure- oder Linolensäurehydroxid enthält, das erhalten wurde durch Reduktion der in einem der Ansprüche 2 bis 4 definierten Produkte.

**6.** Verfahren zur Herstellung eines - oder $\delta$-Lactons der allgemeinen Formel (I), in der R eine lineare gesättigte oder einfach-ungesättigte Alkylkette mit 8 bis 10 Kohlenstoffatomen und $R_1$ Alkylen mit 2 oder 3 Kohlenstoffatomen bedeuten,
das eine Stufe umfaßt, die darin besteht, daß man in einem Substrat, welches das Produkt, ausgewählt aus der Gruppe

- Produkt der Photo-Oxidation der Ölsäure, eines ihrer Ester mit niederen $C_1$-$C_4$-Alkoholen und ihren Glyceriden,
- Produkt der Auto-Oxidation von Fetten, die Ölsäure enthalten, ihrer Ester und ihren Glyceriden, die es enthalten,

einen Mikroorganismus kultiviert (züchtet), der in der Lage ist, die Oxidation des genannten Produkts in $\beta$-Stellung zu bewirken.

**7.** Verfahren nach Anspruch 6, in dem das genannte Substrat ein Ölsäurehydroxid umfaßt, das durch Reduktion der Hydroxide oder Hydroperoxide von Linolsäure und Linolensäure, eines ihrer Ester mit niederen $C_1$-$C_4$-Alkoholen, ihren Glyceriden und ihren Mischungen erhalten wurde.

**8.** Verfahren nach einem der Ansprüche 1 bis 7, bei dem der genannte Mikroorganismus ausgewählt wird aus der Gruppe, die besteht aus Cladosporium suaveolens, Cladosporium cucumberinum, Pichia etchellsii, Sporobolomyces salmonicolor, Candida lipolytica und Fusarium poae, Rhodotorula glutinis, Kloeckera saturnus, Sporobolomyces roseus, Cladosporium capsici, Pichia membranaefaciens, Pichia pamtoris, Hyphopichia burtoni, Kluyveromyces lactis, Aspergillus oryzae, Geotricum klebahnii, Saccharomyces cerevisiae, Saccharomyces delbrueckii und Monilinia fructicola.

**9.** Verfahren nach Anspruch 1 zur Herstellung von (S)-$\delta$-decanoliden, bei dem das genannte Substrat die (S)-13-Hydroxy (oder Hydroperoxy)-cis-9,trans-11-octadecensäure umfaßt und in dem der verwendete Mikroorganismus ausgewählt wird aus der Gruppe, die besteht aus Pichia etchellsii und Cladosporium suaveolens.

**10.** Verfahren nach Anspruch 9, worin das genannte Hydroxid oder Hydroperoxid durch Lipo-Oxidation der Linolsäure mit Soja-Lipooxygenasen hergestellt wurde.

**11.** Verfahren nach einem der Ansprüche 1 bis 10, worin das genannte Hydroxid oder Hydroperoxid in einer optisch aktiven Form vorliegt.

**12.** Verfahren nach einem der Ansprüche 1 bis 11, worin die Rizinolsäure oder ihr Ester dem Substrat zugesetzt wird.

**13.** Verfahren nach Anspruch 12, worin das Verhältnis zwischen dem Substrat und der Menge an Rizinolsäure und ihrem Ester in dem Bereich von 1:2 bis 2:1 liegt.

**14.** Verfahren nach Anspruch 12, worin der Mikroorganismus ausgewählt wird aus der Gruppe, die besteht aus Candida lipolytica und Rhodotorula glutinis.